# EUROPEAN PATENT SPECIFICATION

(11) **EP 3 895 616 B1**
(45) Date of publication and mention of the grant of the patent: **18.11.2026**
(21) Application number: 20465519.5
(22) Date of filing: 15.04.2020
(51) Int. Cl.: A61B 5/18, A61B 5/00, H01S 5/068, H05B 45/10

(54) **METHOD AND DEVICE FOR STEERING INFRARED LIGHT SOURCE, ESPECIALLY FOR EYE SAFETY**
VERFAHREN UND VORRICHTUNG ZUR STEUERUNG EINER INFRAROTLICHTQUELLE, INSBESONDERE FÜR DIE AUGENSICHERHEIT
PROCÉDÉ ET DISPOSITIF PERMETTANT D'ORIENTER UNE SOURCE DE LUMIÈRE INFRAROUGE, NOTAMMENT POUR LA SÉCURITÉ DES YEUX

(43) Date of publication of application: 20.10.2021
(73) Proprietor: AUMOVIO Germany GmbH, 60488 Frankfurt am Main (DE)
(72) Inventor: Lazarciuc, Emil, 65824 Schalbach a. Ts. (DE)
(74) Representative: Aumovio Corporation

(56) References cited:
- WO-A1-2015/108904
- WO-A1-2019/200434
- US-A1- 2004 167 500
- US-A1- 2013 169 155
- US-A1- 2019 372 305
- US-A1- 2020 059 998
- US-B1- 7 742 514

## Description

The present invention relates to an Infrared Light Source Steering Method and Device, especially for eye safety used in Driver Monitoring Systems.

A Driver Monitoring System (DMS), also known as Driver Attention Monitor, is a vehicle safety system that uses infrared sensors to monitor driver attentiveness. The DMS includes an infrared camera which is capable of eye tracking, with the aid of for example infrared LEDs or infrared VCSELs (vertical-cavity surface-emitting laser) for the illumination of the eyes. If the driver is not paying attention to the road ahead and a dangerous situation is detected, the system will warn the driver or take over car control.

Because of different ambiental light conditions encountered, illumination infrared LEDs, infrared VCSELs or other infrared (IR) sources are activated with current pulses having different amplitude and length. In case of improper activation pulse parameters generated by algorithms, system failure or close eye proximity to the IR source, the amount of optical power generated by LEDs, VCSELs, or other light sources emitting in the 800nm to 950nm range, could exceed eye safety limits.

Infrared radiation can cause thermal retina hazard or cornea thermal injury and limits with the given conditions are regulated by IEC 60825-1, IEC 62471 standards for IR-A radiation and European Directive 2006/25/EC.

Cornea exposure limit is defined as the maximum allowed irradiance EIR_max, for different time scales of exposure inside IR-A spectrum. Irradiance EIR is calculated using the inverse square law by considering IR source radiant intensity IIR and distance d from eye: $EIR_max > EIR = IIR / {d}^{∧} 2 \left[{W / m}^{∧}2\right]$ (1)

| | | |
|---|---|---|
| were | IIR | - represents the radiant intensity of the IR source. It is a specific LED or VCSEL parameter function of the electrical current [W/sr], |
| | d | - represents distance from eye of the IR source [m]. |

If the IR source is activated with repetitively pulses, weighted radiant exposure shall be compared with the continuous wave exposure limits by using the time averaged values, tavg_max=0.25[s]: $EIR_max > EIR_avg = IIR_avg / {d}^{∧} 2 = IIR \left(I_LED\right) * {Dc / d}^{∧} 2 \left[{W / m}^{∧}2\right]$ (2) or $IIR_avg = IIR \left(I_LED\right) * Dc = EIR_avg * {d}^{∧} 2 \left[W/sr\right]$ (3)

| | | |
|---|---|---|
| were | Dc | - represents the duty-cycle and is the ratio between pulse width and repetition period: |

$Dc = t_on_OUT / T$ (4)

| | | |
|---|---|---|
| were | t_on_OUT | - represents LED current pulse width [s], |
| | T | - represents LED current pulse repetition period [s]. |

### Example 1:

For an exposure time texp>1000[s] inside the IR-A spectrum, the defined exposure limit is EIR_max=100[W/m^2]. Using SFH 4770S an infrared LED with a typical radiant intensity IIR_typ(1.5A)=560[mW/sr], the current value I_LED=1.5[A] and the eye distance d=0.1[m], calculated irradiance EIR, according equation (1), is 0.56[W/sr]/0.1[m^2]=56 [W/m^2], which is below the limit.

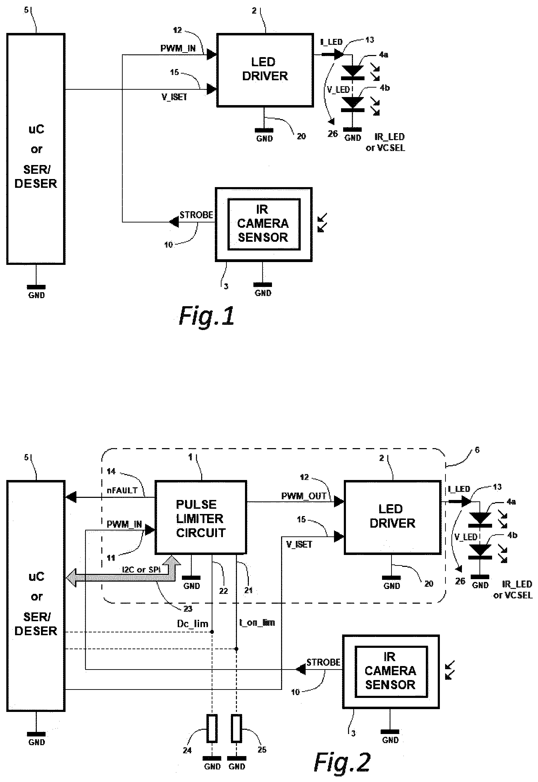

A typical implementation of a Prior Art DMS is presented in Fig.1 and comprises an infrared camera sensor 3 which controls an LED driver 2 using an activation signal, named STROBE 10.

LED current I_LED 13 of IR LEDs or VCSELs 4a, 4b is set by V_ISET voltage 16, generated by a micro-controller or a ser/deser circuit 5, according to equation: $I _ LED = gdrv * V _ ISET \left[A\right]$ (5)

| | | |
|---|---|---|
| were | I_LED | - represents the current into IR LEDs or VCSELs [A], |
| | gdrv | - represents the transconductance of the LED Driver [A/V], |
| | V_ISET | - represents the current set voltage [V]. |

Assuming a linear relationship between I_LED current and radiant intensity, $IIR \left(I_LED\right) = kI * I_LED \left[W/sr\right]$ (6)

| | | |
|---|---|---|
| were | kl | - represents LED current conversion coefficient [V/sr], |

using equation (5): $IIR \left(I_LED\right) = kI * gdrv * V _ ISET \left[W/sr\right]$ (7)

During global shutter time frame, camera sensor STROBE pulse width is adapted to ambiental lighting conditions. In case of improper STROBE pulse parameters generated by algorithms or system failure or close eye proximity to IR source, the amount of optical irradiance could exceed eye safety limits.

WO 2015/108904 A1 relates to methods and apparatuses for controlling intensity of illumination in eye based biometric systems, for achieving one or both of eye safety or power efficiencies in biometric systems that rely on distinctive characteristics of the eye (such as iris based biometrics or retina based biometrics). US 2019/372305 A1 relates to the technical field of consumer electronics, and more particularly to a control system and method for a laser projector, and a terminal. WO 2019/200434 A1 relates to a control system and in particular to a control system for one or more infrared light sources. US 7 742 514 B1 relates generally to optical navigation sensors. US 2004/167500 A1 relates to a dermatologic treatment apparatus and method, and particularly to a light-based, self-contained, cordless and hand-held apparatus. US 2020/059998 A1 generally describes a system and a method for monitoring an occupant in a vehicle. The system may include an illuminator, such as an infrared light emitting diode (IR LED), and a detection device, such as a camera. US 2013/169155 A1 relates to a headlamp device for a vehicle, and more particularly, to a headlamp device with adjustable brightness and a luminance control method for the headlamp device.

An object of the present invention is to provide an improved method and device for infrared light source irradiance limit for providing safety for the eyes, i.e. that the eyes are not injured by the IR LED pulses in any way.

This object is achieved by a method according claim 1 and a device according claim 11. The dependent claims achieve further improvements of the invention.

By comparing the requested current pulses with the maximum allowed given limit and reshaping those current pulses which would exceed the given limit it is assured that a human eye exposed to the infrared light source will not receive a critical amount of infrared light from the infrared light source. Reshaping means limiting the pulse width and /or the pulse amplitude sent out from the infrared light source. So single or several pulses can be suppressed totally or cut in pulse width and/ or pulse amplitude even if requested for a required illumination intensity of a device, in which the invention is used, would request an amount of infrared light critical for the human eye.

By feeding the current using a function with rising and falling edges between a low level and a high level, whereby a current is send to the LED only when there is requested a current and depending of the application the function is on a rising edge respective high-level or a falling edge respective low level of a constant function is one possible solution to obtain the safety of the human eye. Thus, it is assured that the amount of infrared light sent out to the human eye will not be harmful to the human eye.

By feeding the current only on high-level of the constant function it is assured that the infrared light source does not emit infrared light without interruption.

If the constant function depends from the maximum allowed infrared energy for the human eye correlated with the distance between the human eye and the infrared light source, it is assured that the amount of infrared light sent to the human eye is in a safe condition.

If the distance between the human eye and the infrared light source is measured it is assured that under every circumstance, that the amount of infrared light sent from the infrared light source to the human eye is in a safe scope.

The invention is suitable for example for use in a driver monitoring system and/or or a cabin monitoring system, since especially during the use of such systems in night time an illumination which will not distract or hinder a driver of a vehicle is required for the use of one or more cameras used by these systems.

By a device for steering an infrared light source comprising a light sensor, whereby the device executes one or more methods of the invention it is received a device assuring the eye safety of a possible user of such an infrared light source.

If the device comprises a sensor for measuring the distance between the human eye and the infrared light source it is assured that the amount of light sent to the human eye is safe under every condition, even if a user is nearer to the infrared light source than expected by the designers of such a system.

If the device according to the invention comprises a pulse limiter circuit (1) comprising analog and logical circuits and is based on a timing capacitor charge/discharge method a good designable and steerable device can be reached.

If the device comprises a pulse limiter comprising digital circuits the inventive method can be executed in digital form using software as will be demonstrated more in detail for a possible solution later.

If the infrared light source comprises one or more LEDs a functional and durable light source is constructed.

If the infrared light source comprises one or more VCSELs a low power consuming light source is created.

If a proximity sensor for measuring the distance between human eye and infrared light source is present the security for the eyes can be received in every situation.

If the proximity sensor is a proximity infrared sensor the illumination of the infrared light source can be used.

The invention will now be described exemplary for different solutions based on the Figures.
Fig. 2 shows a first embodiment of the invention.
Fig. 3 shows a second embodiment of the invention.
Fig. 4 shows timing diagrams of LEDs average current conditioned by duty-cycle limit and current set voltage reference limit.

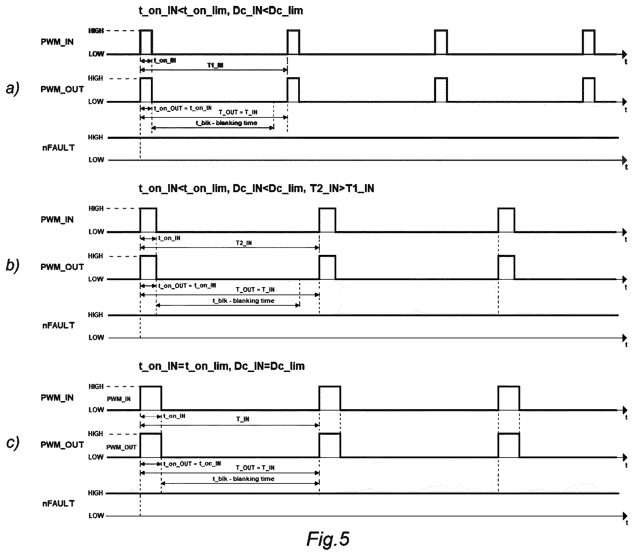

Fig. 5 shows timing diagrams of the pulses based on described method for normal operating parameters a), b) and c) cases.

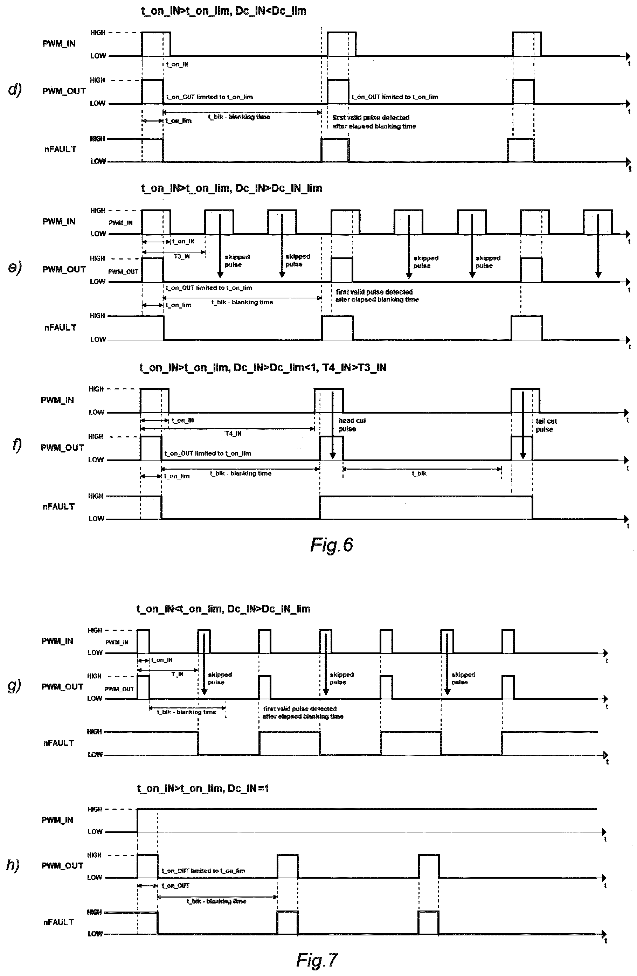

Fig. 6 shows timing diagrams of the pulses based on described method for out of range operating parameters d), e) and f) cases.
Fig. 7 shows timing diagrams of the pulses based on described method for out of range operating parameters g) and h) cases.
Fig. 8 shows a first implementation of a pulse limiter using analog and logical circuits.
Fig. 9 shows a timing diagram of implemented device for different pulse conditions.
Fig. 10 shows a logic diagram of a second embodiment of another implementation variant.

A first embodiment of the invention in Fig.2 refers to a method and device for DMS camera eye safety optical irradiance limit by LED driver circuit parameters monitor and limit to avoid driver eye hazard, thus limit average optical irradiance of infrared LEDs or VCSELs as part of DMS implementation for ASIL-A and ASIL-B compliance.

Parameters monitoring refers to STROBE pulse monitoring. Pulse limiter circuit 1 can be implemented as a standalone or embedded 6 into the LED driver circuit 2.

In Fig. 2 the first embodiment of the invention further comprises a micro-processing unit or ser/deser circuit 5, according to DMS architecture, an infrared camera sensor 3, an LED driver 2, and one or several IR LEDs or VCSELs 4a, 4b. A STROBE pulse 10 from the infrared camera sensor 3 activates the LED driver 2 via pulse limiter circuit 1. LEDs current 13 amplitude is set via V_ISET voltage 15 and timing parameters are limited for PWM_OUT signal 12.

Pulse limiter circuit 1 features a logic level input PWM_IN 11, a logic level output PWM_OUT 12, an open drain active low fault reporting output nFAULT 14 and two set pins 21 and 22 for on-time and duty-cycle limit setting using external resistors 24 and 25 or voltage references or a serial port 23, I2C or SPI.

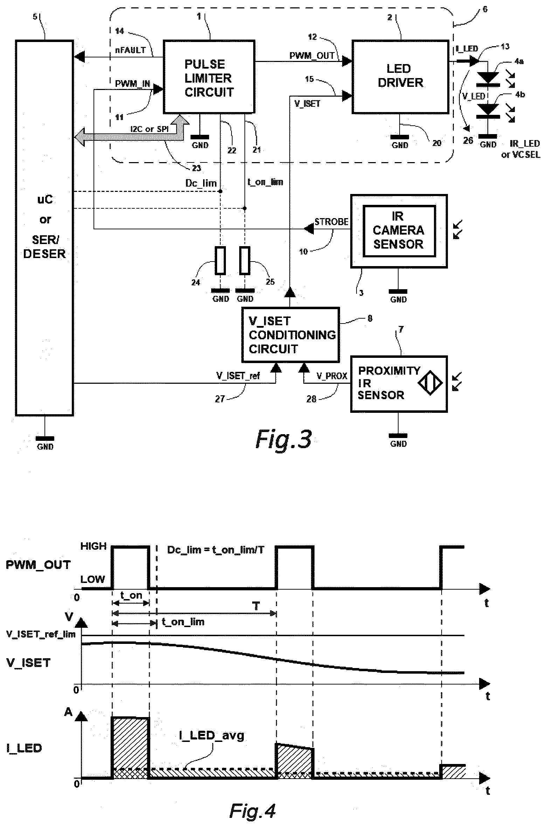

A second embodiment of the invention in Fig 3 refers to a method and device for DMS camera eye safety optical irradiance limit by LED driver circuit parameters monitor and limit to avoid driver eye hazard, thus limit average optical irradiance of infrared LEDs or VCSELs as part of DMS implementation for ASIL-A and ASIL-B compliance.

The second embodiment of the invention, shown in Fig.3, increases further eye safety level by correlating LEDs current with eye proximity information using a conditioning circuit 8 to modulate the current set reference voltage V_ISET_ref 27 with a proximity sensor 7 output modulation voltage V_PROX 28.

Current set reference voltage V_ISET_ref is as well limited to a limit value V_ISET_ref_lim inside the conditioning circuit by using voltage limiting components like Zenner diodes or shunt regulators. The LEDs current, set by V_ISET 15, should follow the same square law as radiant intensity from equation (3): $V _ ISET = kv * V _ ISET _ ref * {\left(V_PROX\right)}^{∧} 2 \left[V\right]$ (8)

| | | |
|---|---|---|
| were | V_ISET | - represents LEDs current set voltage [V], |
| | kv | - represents conditioning circuit conversion coefficient, implementation specific [1/V^2], |
| V_ISET_ref | | - represents current set voltage reference [V], |
| V_PROX | | - proximity sensor output voltage [V]: |

$V_PROX = km * d \left[V\right]$ (9)

| | | |
|---|---|---|
| were | km | - represents proximity sensor conversion coefficient [V/m], |
| | d | - represents distance between the eye and the IR source [m]. |

By putting together equations (7), (8) and (9), radiant intensity can be written: $IIR \left(I_LED\right) = kI * gdrv * kv * V_ISET_ref * {\left(km*d\right)}^{∧} 2 \left[W/sr\right]$ (10)

The average optical irradiance, based on equation (2), becomes: $EIR _ avg = kI * gdrv * kv * {km}^{∧} 2 * Dc * V _ ISET _ ref \left[W/{m}^{∧}2\right]$ (11)

Average optical irradiance depends on the current set reference voltage and PWM_OUT duty-cycle with a proportionality conversion coefficient: $EIR _ avg = kE * Dc * V _ ISET _ ref \left[W/{m}^{∧}2\right]$ (12) $kE = kI * gdrv * kv * {km}^{∧} 2 \left[A/{m}^{∧}2\right]$ (13)

| | | |
|---|---|---|
| were | kE | - represents the proportionality conversion coefficient of the device having a design specific value [A/m^2]. |

By limiting the PWM_OUT duty-cycle and current set voltage reference V_ISET_ref, maximum average current thus maximum average irradiance level is limited to a value below maximum allowed irradiance limit assuring the requested eye safety level: $EIR_avg_lim = kE * Dc_lim * V_ISET_ref_lim < EIR_max \left[W/{m}^{∧}2\right]$ (14)

| | | |
|---|---|---|
| were | Dc_lim | - represents duty-cycle limit, |
| | V_ISET_ref_lim | - represents the current set reference voltage limit [V], |
| | EIR_max | - represents maximum allowed irradiance limit [W/m^2]. |

The way how the average LEDs current is limited by keeping V_ISET below V_ISET_ref_lim and PWM_OUT duty-cycle below Dc_lim is presented in Fig.4. The device input signal PWM_IN 11 (STROBE 10) is monitored by checking the on-time t_on_IN and duty-cycle Dc_IN. Limiting conditions of t_on_lim and Dc_lim are applied to PWM_OUT 12 by limit the t_on_OUT and Dc_OUT.

Pulse parameters are defined as follows:
- t_on_IN: - PWM input pulse on-time [s],
- T_IN: - PWM input pulse period [s],
- Dc_IN: - PWM input pulse duty-cycle,
- t_on_OUT: - PWM output pulse on-time [s],
- T_OUT: - PWM output pulse period [s],
- Dc_OUT: - PWM output pulse duty-cycle,
- t_on_lim: - PWM pulse on-time limit [s],
- Dc_lim: - PWM duty-cycle limit,
- t_blk: - Blanking time [s].

If input pulse on time and/or duty-cycle exceeds the set limits a fault condition will be reported by activating nFAULT and output pulses are reshaped according to following condition/s: $IF t_on_IN > t_on_lim THEN t_on_OUT = t_on_lim ELSE t_on_OUT = t_on_IN$ (15)

In this way, the PWM output pulse on time is limited to t_on_lim, based on IR LED thermal management considerations.

A blanking time is generated based on the duty-cycle limit set value: $t _ blk = t _ on _ OUT * \left(1-Dc_lim\right) / Dc _ lim \left[s\right]$ (16)

During blanking time, all pulses from PWM_IN are ignored thus the corresponding PWM_OUT pulse or pulses are skipped or cut. The first valid pulse after elapsed blanking time is considered.

In this way, the PWM output pulse duty-cycle is limited to Dc_lim and the IR LED average radiant intensity limited to Dc_lim of pulse peak radiant in intensity. If the PWM frequency is increased but t_on_IN<t_on_lim AND Dc_IN<Dc-lim, input pulses are reproduced at the output and no-fault condition is reported.

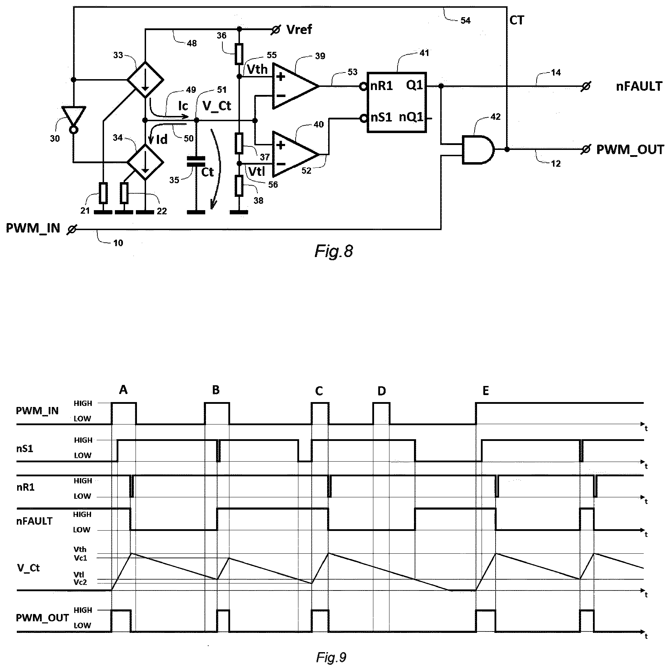

In Fig. 8 a first implementation of such pulse limiter uses analog and logical circuits and is based on a timing capacitor charge/discharge method using different charge/discharge current set values between two voltage threshold levels. The circuit shown in Fig.8 comprises a controlled constant current source circuit 33 with the charge current Ic 49 set by resistor 21, a controlled constant current sink circuit 34 with the discharge current Id 50 set by resistor 22, a timing capacitor Ct, a window comparator circuit using comparator circuits 39 and 40 using reference voltages Vth 55 and Vtl 56 generated by a voltage reference Vref 48 divided by resistors 36, 36 and 38, an inverted inputs RS flip-flop 41, an AND gate logical circuit 42 which passes through or blocks the PWM_IN 10 pulses to the output PWM_OUT 12 according to nFAULT logic level, a logical inverter circuit 30 controls the constant current circuits according to signal CT 54 logic level.

During PWM_IN pulse on-time, the timing capacitor is charged with a charge current Ic. If PWM_IN pulse on-time is higher than the capacitor charge time to the high-level threshold voltage Vth 55 then RS flip-flop 41 is reset and gate 42 will cut remaining part of the pulse at PWM_OUT.

In this way, the circuit implements the on-time limit function.

Then timing capacitor is discharged with a discharge current Id until capacitor voltage V_Ct 51 decreases below low-level threshold voltage Vtl 56. Discharge time is called also blanking time because the gate is locked and no PWM_IN pulse passes through. After elapsed blanking time, RS flip-flop 41 is set and gate 42 is unlocked. If no valid pulse is present at PWM_IN, timing capacitor is discharged completely, and another monitoring cycle can be started.

In this way, the circuit implements the off-time limit function.

Timing capacitor charge time between two voltage levels is given by the equation: $tch = Ct * \left(Vth-Vtl\right) / Ic \left[s\right]$ (17)

| | | |
|---|---|---|
| were | Ct | - represents timing capacitor value [F], |
| | Vth | - represents high-level threshold voltage value [V], |
| | Vtl | - represents low-level threshold voltage value [V], |
| | Ic | - represents current source charge current [A]. |

Timing capacitor discharge time or blanking time is given by: $tblk = Ct * \left(Vth-Vtl\right) / Id \left[s\right]$ (18)

| | | |
|---|---|---|
| were | Id | - represents current sink discharge current [A]. |

Duty-cycle limit function is implemented using on-time and off-time limit functions: $Dc_lim = t_on_lim / \left(t_on_lim+t_blk\right) = Id / \left(Ic+Id\right)$ (19)

### Example 2:

If charge current Ic=25[uA], discharge current Id=8.33[uA], timing capacitor value Ct=100[nF], high-level threshold voltage Vth=1.1[V] and low-level threshold voltage Vtl=0.1[V], calculated capacitor charge time is tch=4[ms], discharge time is tblk=12[ms] and the duty-cycle limit is Dc_lim=0.25.

If timing capacitor is charged from zero and discharged completely, duty-cycle limited value remains unchanged.

Fig. 9 shows a timing diagram of the implemented device shown in Fig. 8 for different pulse conditions:
Pulse A on-time is higher than on-time limit and duty-cycle higher than duty-cycle limit so part of it is cut. Pulse B on-time is equal with on-time limit and duty-cycle lower than duty-cycle limit but arrives during pulse A blanking time thus part of it is cut.
Pulse C on-time is smaller than on-time limit, but the duty-cycle is higher than the duty-cycle limit and pulse D is cut completely because arrives during pulse C blanking time.
Pulse D on-time is much higher than on-time limit and the duty-cycle is much higher than duty-cycle limit, thus pulse on-time is limited to on-time limit and duty-cycle is limited to duty-cycle limit.

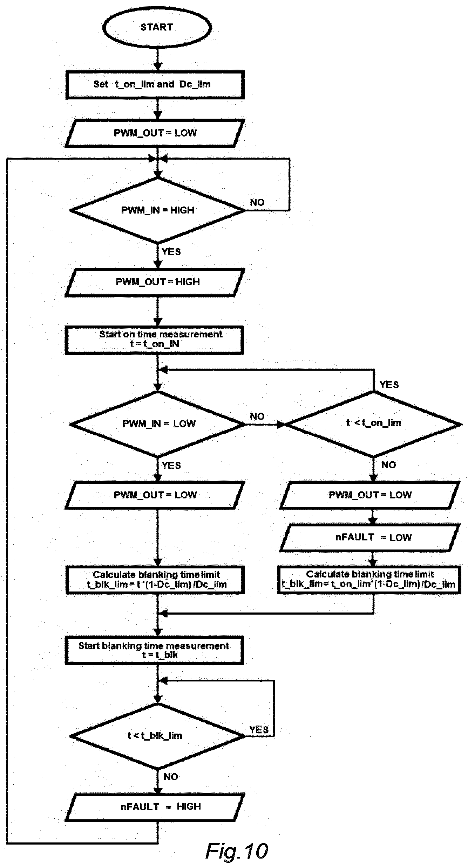

Another implementation variant comprises a microcontroller by using a GPIO timing port for PWM_IN and two GPIO ports for PWM_OUT and fault reporting nFAULT output. The logic diagram of such an embodiment is shown in Fig.10. A monitoring cycle is composed by two sequences: on-time monitoring sequence and blanking time sequence.

At startup, the device is initialized by setting the on-time limit, duty-cycle limit. PWM output is set to LOW logic level.

When PWM_IN input goes HIGH then PWM_OUT output is set to HIGH logic level and the device starts the on-time monitoring sequence by measuring the PWM_IN pulse on-time. If measured on-time is smaller than the on-time limit, PWM_OUT output is set to LOW logic level and the blanking time is calculated based on duty-cycle limit. If PWM_IN input stays on HIGH logic level more than the on-time limit, PWM_OUT output is set to LOW logic level, nFAULT output is set to LOW logic level (active) and the blanking time is calculated based on on-time and duty-cycle limits.

Next, device starts the blanking time sequence. During blanking time, all PWM_IN HIGH logic levels are ignored thus corresponding pulse or pulses are cut. After elapsed blanking time, nFAULT output is set to HIGH logic level (inactive). Next the device checks the PWM_IN input for another valid HIGH logic level and a new monitoring cycle begins.

The invention provides the following advantages:
- The Eye safety function is assured by limiting the optical average irradiance for IR LEDs or VCSELs or other infrared sources
- The limited optical average irradiance by limiting the duty-cycle value
- The limited optical average radiant intensity by correlating the I_LED current with proximity sensor information
- LEDs or VCSELs damage mitigation by on-time limit set, application specific, based on thermal management conditions
- Parameter limits set by Hardware or Software
- Operate independent of camera sensor framerate variation
- Fault reporting output
- ASIL-A and ASIL-B compliant

The invention can be used in a wide range of applications. Examples are:
- Machine vision
- Consumer products
- Military
- Avionics
- Applications where high irradiance IR pulses are used

## Claims

1. A method for steering an infrared light source (4a, 4b), the light source (4a, 4b) being fed by current pulses (13) to emit light pulses and the current pulses (13) being requested according to a present illumination situation, the method comprising:
- comparing the requested current pulses with a maximum allowed given limit;
- reshaping those current pulses which would exceed the maximum allowed given limit;
- performing a duty-cycle limit function, the duty-cycle limit function being implemented by an on-time limit function and an off-time limit function;
**characterized in that** the off-time limit function is performed by generating a blanking time (t_blk) based on a duty cycle limit set value (Dc_lim) that is provided by a micro-controller or a ser/deser circuit (5) to a pulse limiter circuit (1), wherein during the blanking time, all pulses (PWM_IN) provided by an infrared camera sensor (3) to the pulse limiter circuit (1) are ignored such that corresponding pulses (PWM_OUT) provided by the pulse limiter circuit (1) to a driver (2) of the infrared light source (4a, 4b) are skipped or cut.

2. The method according to claim 1, wherein the infrared light source (4a, 4b) is fed by a current (13) using a function with rising and falling edges between a low level and a high level, wherein a current is sent to the infrared light source (4a, 4b) only when a current is requested and, depending on the application, when the function is on a rising edge respective high-level or a falling edge respective low level of a constant function.

3. The method according to claim 2, wherein the light source (4a, 4b) is fed by the current only on high-level of the constant function.

4. The method according to claim 2, wherein the constant function is dependent on a maximum allowed infrared energy for the human eye correlated with a distance between the human eye and the infrared light source (4a, 4b).

5. The method according to claim 4, further comprising measuring the distance between the human eye and the infrared light source (4a, 4b).

6. The method according to one of the preceding claims, wherein the method is used in a driver monitoring system or cabin monitoring system.

7. The method according to one of the preceding claims, further comprising performing the on-time limit function by comparing an on-time (t_on_IN) of the pulses (PWM_IN) provided by the infrared camera sensor (3) to the pulse limiter circuit (1) with an on-time limit (t_on_lim), and if the pulse on-time (t_on_IN) is larger than the on-time limit (t_on_lim), limiting an on-time (t_on_OUT) of the pulses (PWM_OUT) provided by the pulse limiter circuit (1) to the driver (2) of the infrared light source (4a, 4b) to the on-time limit (t_on_lim).

8. The method according to one of the preceding claims, further comprising performing the duty-cycle limit function by using on-time and off-time limit functions such that a duty cycle of the pulses (PWM_OUT) provided by the pulse limiter circuit (1) to the driver (2) of the infrared light source (4a, 4b) is limited to a duty-cycle limit (Dc_lim).

9. The method according to one of the preceding claims, further comprising performing the on-time limit function by charging a timing capacitor during an on-time of the pulses (PWM_IN) provided by the infrared camera sensor (3) to the pulse limiter circuit (1) with a charge current (Ic), and if the on-time is larger than a capacitor charge time to a high-level threshold voltage (Vth, 55), resetting an RS flip-flop (41) and cutting a remaining part of the pulse (PWM_OUT) provided by the pulse limiter circuit (1) to the driver (2) of the infrared light source (4a, 4b) by a gate (42).

10. The method according to one of the preceding claims, further comprising performing the off-time limit function by discharging a timing capacitor with a discharge current (Id) until a capacitor voltage (V_Ct, 51) decreases below a low-level threshold voltage (Vtl, 56) and, after a blanking time elapses, setting an RS flip-flop (41) and unlocking a gate (42) and, if no valid pulse (PWM_IN) is present at an input of the pulse limiter circuit (1), discharging the timing capacitor completely.

11. A device for steering an infrared light source comprising a light sensor (3), a micro-controller or a ser/deser circuit (5), a pulse limiter circuit (1) and driver (2), wherein the device is configured to execute a method according one of claims 1 to 10.

12. The device according to claim 11, wherein the device comprises a sensor (7) for measuring a distance between a human eye and an infrared light source.

13. The device according to claim 11 or 12, wherein the pulse limiter circuit (1) is comprising analog and logical circuits and is based on a timing capacitor charge/discharge method.

14. The device according to claim 11 or 12, wherein the pulse limiter circuit (1) is comprising digital circuits.

15. The device according to one of claims 11 to 14, wherein the infrared light source (4a, 4b) comprises one or more LEDs.

16. The device according to one of claims 11 to 14, wherein the infrared light source (4a, 4b) comprises one or more VCSELs.

17. The device according to claim 12, wherein the sensor (7) is a proximity IR sensor.

## Patentansprüche

1. Verfahren zur Steuerung einer Infrarotlichtquelle (4a, 4b), wobei die Lichtquelle (4a, 4b) durch Strompulse (13) gespeist wird, um Lichtpulse zu emittieren, und die Strompulse (13) gemäß einer aktuellen Beleuchtungssituation angefordert werden, wobei das Verfahren Folgendes umfasst:
- Vergleichen der angeforderten Strompulse mit einem maximal zulässigen gegebenen Grenzwert;
- Umformen derjenigen Strompulse, die den maximal zulässigen gegebenen Grenzwert überschreiten würden;
- Durchführen einer Tastverhältnisgrenzwertfunktion, wobei die Tastverhältnisgrenzwertfunktion durch eine Einschaltzeitgrenzwertfunktion und eine Ausschaltzeitgrenzwertfunktion implementiert wird;
**dadurch gekennzeichnet, dass** die Ausschaltzeitgrenzwertfunktion durch Generieren einer Austastzeit (t_blk) basierend auf einem Tastverhältnisgrenzwerteinstellungswert (Dc_lim) durchgeführt wird, der durch eine Mikrosteuereinheit oder eine SerDes-Schaltung (5) an eine Pulsbegrenzungsschaltung (1) bereitgestellt wird, wobei während der Austastzeit alle Pulse (PWM_IN), die durch einen Infrarotkamerasensor (3) an die Pulsbegrenzungsschaltung (1) bereitgestellt werden, derart ignoriert werden, dass entsprechende Pulse (PWM_OUT), die durch die Pulsbegrenzungsschaltung (1) an einen Treiber (2) der Infrarotlichtquelle (4a, 4b) bereitgestellt werden, übersprungen oder abgeschnitten werden.

2. Verfahren nach Anspruch 1, wobei die Infrarotlichtquelle (4a, 4b) durch einen Strom (13) unter Verwendung einer Funktion mit ansteigenden und abfallenden Flanken zwischen einem niedrigen Pegel und einem hohen Pegel gespeist wird, wobei ein Strom nur dann an die Infrarotlichtquelle (4a, 4b) gesendet wird, wenn ein Strom angefordert wird und, abhängig von der Anwendung, wenn sich die Funktion an einem jeweiligen hohen Pegel der ansteigenden Flanke oder an einem jeweiligen niedrigen Pegel der abfallenden Flanke einer konstanten Funktion befindet.

3. Verfahren nach Anspruch 2, wobei die Lichtquelle (4a, 4b) nur auf hohem Pegel der konstanten Funktion durch den Strom gespeist wird.

4. Verfahren nach Anspruch 2, wobei die konstante Funktion von einer maximal zulässigen Infrarotenergie für das menschliche Auge abhängig ist, die mit einem Abstand zwischen dem menschlichen Auge und der Infrarotlichtquelle (4a, 4b) korreliert ist.

5. Verfahren nach Anspruch 4, ferner umfassend Messen des Abstands zwischen dem menschlichen Auge und der Infrarotlichtquelle (4a, 4b).

6. Verfahren nach einem der vorhergehenden Ansprüche, wobei das Verfahren in einem Fahrerüberwachungssystem oder einem Kabinenüberwachungssystem verwendet wird.

7. Verfahren nach einem der vorhergehenden Ansprüche, ferner umfassend Durchführen der Einschaltzeitgrenzwertfunktion durch Vergleichen einer Einschaltzeit (t_on_IN) der durch den Infrarotkamerasensor (3) an die Pulsbegrenzungsschaltung (1) bereitgestellten Pulse (PWM_IN) mit einem Einschaltzeitgrenzwert (t_on_lim) und, falls die Pulseinschaltzeit (t_on_IN) größer als der Einschaltzeitgrenzwert (t_on_lim) ist, Begrenzen einer Einschaltzeit (t_on_OUT) der durch die Pulsbegrenzungsschaltung (1) an den Treiber (2) der Infrarotlichtquelle (4a, 4b) bereitgestellten Pulse (PWM_OUT) auf den Einschaltzeitgrenzwert (t_on_lim).

8. Verfahren nach einem der vorhergehenden Ansprüche, ferner umfassend Durchführen der Tastverhältnisgrenzwertfunktion durch Verwenden von Einschaltzeit- und Ausschaltzeitgrenzwertfunktionen derart, dass ein Tastverhältnis der durch die Pulsbegrenzungsschaltung (1) an den Treiber (2) der Infrarotlichtquelle (4a, 4b) bereitgestellten Pulse (PWM_OUT) auf einen Tastverhältnisgrenzwert (Dc_lim) begrenzt wird.

9. Verfahren nach einem der vorhergehenden Ansprüche, ferner umfassend Durchführen der Einschaltzeitgrenzwertfunktion durch Laden eines Zeitgeberkondensators während einer Einschaltzeit der durch den Infrarotkamerasensor (3) an die Pulsbegrenzungsschaltung (1) bereitgestellten Pulse (PWM_IN) mit einem Ladestrom (Ic) und, falls die Einschaltzeit größer als eine Ladezeit des Kondensators bis zu einer Schwellenspannung mit hohem Pegel (Vth, 55) ist, Zurücksetzen eines RS-Flipflops (41) und Abschneiden eines verbleibenden Teils des durch die Pulsbegrenzungsschaltung (1) an den Treiber (2) der Infrarotlichtquelle (4a, 4b) bereitgestellten Pulses (PWM_OUT) durch ein Gate (42).

10. Verfahren nach einem der vorhergehenden Ansprüche, ferner umfassend Durchführen der Ausschaltzeitgrenzwertfunktion durch Entladen eines Zeitgeberkondensators mit einem Entladestrom (Id), bis eine Kondensatorspannung (V_Ct, 51) unter eine Schwellenspannung mit niedrigem Pegel (Vtl, 56) sinkt, und nach Ablauf einer Austastzeit Setzen eines RS-Flipflops (41) und Entsperren eines Gates (42) und, falls kein gültiger Puls (PWM_IN) an einem Eingang der Pulsbegrenzungsschaltung (1) anliegt, vollständiges Entladen des Zeitgeberkondensators.

11. Vorrichtung zur Steuerung einer Infrarotlichtquelle, umfassend einen Lichtsensor (3), eine Mikrosteuereinheit oder eine SerDes-Schaltung (5), eine Pulsbegrenzungsschaltung (1) und einen Treiber (2), wobei die Vorrichtung konfiguriert ist, um ein Verfahren nach einem der Ansprüche 1 bis 10 auszuführen.

12. Vorrichtung nach Anspruch 11, wobei die Vorrichtung einen Sensor (7) zum Messen eines Abstands zwischen einem menschlichen Auge und einer Infrarotlichtquelle umfasst.

13. Vorrichtung nach Anspruch 11 oder 12, wobei die Pulsbegrenzungsschaltung (1) analoge und logische Schaltungen umfasst und auf einem Zeitgeberkondensatorlade-/entladeverfahren basiert.

14. Vorrichtung nach Anspruch 11 oder 12, wobei die Pulsbegrenzungsschaltung (1) digitale Schaltungen umfasst.

15. Vorrichtung nach einem der Ansprüche 11 bis 14, wobei die Infrarotlichtquelle (4a, 4b) eine oder mehrere LED umfasst.

16. Vorrichtung nach einem der Ansprüche 11 bis 14, wobei die Infrarotlichtquelle (4a, 4b) einen oder mehrere VCSEL umfasst.

17. Vorrichtung nach Anspruch 12, wobei der Sensor (7) ein Näherungs-IR-Sensor ist.

## Revendications

1. Procédé permettant d'orienter une source de lumière infrarouge (4a, 4b), la source de lumière (4a, 4b) étant alimentée par des impulsions de courant (13) pour émettre des impulsions lumineuses et les impulsions de courant (13) étant demandées selon une situation d'éclairage actuelle, le procédé comprenant :
- la comparaison des impulsions de courant demandées avec une limite maximale autorisée donnée ;
- le remodelage des impulsions de courant qui dépasseraient la limite maximale autorisée ;
- l'exécution d'une fonction de limitation de cycle de service, la fonction de limitation de cycle de service étant mise en œuvre par une fonction de limitation de durée d'activation et une fonction de limitation de durée de désactivation ;
**caractérisé en ce que** la fonction de limitation de durée de désactivation est exécutée en générant une période de suppression (t_blk) basée sur une valeur définie de limitation de cycle de service (Dc_lim) fournie par un micro-contrôleur ou un circuit ser/deser (5) à un circuit limiteur d'impulsions (1), dans lequel, pendant la période de suppression, toutes les impulsions (PWM_IN) fournies par un capteur de caméra infrarouge (3) au circuit limiteur d'impulsions (1) sont ignorées de sorte que les impulsions (PWM_OUT) correspondantes fournies par le circuit limiteur d'impulsions (1) à un pilote (2) de la source de lumière infrarouge (4a, 4b) sont ignorées ou coupées.

2. Procédé selon la revendication 1, dans lequel la source de lumière infrarouge (4a, 4b) est alimentée par un courant (13) utilisant une fonction avec des fronts montants et descendants entre un niveau bas et un niveau haut, dans lequel un courant est envoyé à la source de lumière infrarouge (4a, 4b) uniquement lorsqu'un courant est demandé et, selon l'application, lorsque la fonction est sur un front montant par rapport à un niveau haut ou sur un front descendant par rapport à un niveau bas d'une fonction constante.

3. Procédé selon la revendication 2, dans lequel la source de lumière (4a, 4b) est alimentée par le courant uniquement au niveau haut de la fonction constante.

4. Procédé selon la revendication 2, dans lequel la fonction constante dépend d'une énergie infrarouge maximale autorisée pour l'œil humain, corrélée à une distance entre l'œil humain et la source de lumière infrarouge (4a, 4b).

5. Procédé selon la revendication 4, comprenant en outre la mesure de la distance entre l'œil humain et la source de lumière infrarouge (4a, 4b).

6. Procédé selon l'une des revendications précédentes, dans lequel le procédé est utilisé dans un système de surveillance de pilote ou un système de surveillance de cabine.

7. Procédé selon l'une des revendications précédentes, comprenant en outre l'exécution de la fonction de limitation de durée d'activation en comparant une durée d'activation (t_on_IN) des impulsions (PWM_IN) fournies par le capteur de caméra infrarouge (3) au circuit limiteur d'impulsions (1) avec une limite de durée d'activation (t_on_lim), et si la durée d'activation d'impulsion (t_on_IN) est supérieure à la limite de durée d'activation (t_on_lim), la limitation d'une durée d'activation (t_on_OUT) des impulsions (PWM_OUT) fournies par le circuit limiteur d'impulsions (1) au pilote (2) de la source de lumière infrarouge (4a, 4b) à la limite de durée d'activation (t_on_lim).

8. Procédé selon l'une des revendications précédentes, comprenant en outre l'exécution de la fonction de limitation de cycle de service en utilisant des fonctions de limitation de durée d'activation et de durée de désactivation de sorte qu'un cycle de service des impulsions (PWM_OUT) fournies par le circuit limiteur d'impulsions (1) au pilote (2) de la source de lumière infrarouge (4a, 4b) est limité à une limite de cycle de service (Dc_lim).

9. Procédé selon l'une des revendications précédentes, comprenant en outre l'exécution de la fonction de limitation de durée d'activation en chargeant un condensateur de temporisation pendant une durée d'activation des impulsions (PWM_IN) fournies par le capteur de caméra infrarouge (3) au circuit limiteur d'impulsions (1) avec un courant de charge (Ic), et si la durée d'activation est supérieure à une durée de charge de condensateur à une tension de seuil de niveau haut (Vth, 55), la réinitialisation d'une bascule RS (41) et la coupure d'une partie restante de l'impulsion (PWM_OUT) fournie par le circuit limiteur d'impulsions (1) au pilote (2) de la source de lumière infrarouge (4a, 4b) par une porte (42).

10. Procédé selon l'une des revendications précédentes, comprenant en outre l'exécution de la fonction de limitation de durée de désactivation en déchargeant un condensateur de temporisation avec un courant de décharge (Id) jusqu'à ce qu'une tension de condensateur (V_Ct, 51) passe en dessous d'une tension de seuil de niveau bas (Vtl, 56) et, après l'écoulement d'une période de suppression, la configuration d'une bascule RS (41) et le déverrouillage d'une porte (42) et, si aucune impulsion valide (PWM_IN) n'est présente à une entrée du circuit limiteur d'impulsions (1), le déchargement complet du condensateur de temporisation.

11. Dispositif permettant d'orienter une source de lumière infrarouge comprenant un capteur de lumière (3), un micro-contrôleur ou un circuit ser/deser (5), un circuit limiteur d'impulsions (1) et un pilote (2), dans lequel le dispositif est configuré pour exécuter un procédé selon l'une des revendications 1 à 10.

12. Dispositif selon la revendication 11, dans lequel le dispositif comprend un capteur (7) permettant de mesurer une distance entre un œil humain et une source de lumière infrarouge.

13. Dispositif selon la revendication 11 ou 12, dans lequel le circuit limiteur d'impulsions (1) comprend des circuits analogiques et logiques et est basé sur un procédé de charge/décharge de condensateur de temporisation.

14. Dispositif selon la revendication 11 ou 12, dans lequel le circuit limiteur d'impulsions (1) comprend des circuits numériques.

15. Dispositif selon l'une des revendications 11 à 14, dans lequel la source de lumière infrarouge (4a, 4b) comprend une ou plusieurs LED.

16. Dispositif selon l'une des revendications 11 à 14, dans lequel la source de lumière infrarouge (4a, 4b) comprend un ou plusieurs VCSEL.

17. Dispositif selon la revendication 12, dans lequel le capteur (7) est un capteur IR de proximité.
